# EUROPEAN PATENT APPLICATION

(11) **EP 4 715 370 A1**
(43) Date of publication of application: **25.03.2026**
(21) Application number: 24808527.6
(22) Date of filing: 31.01.2024
(51) Int. Cl.: G01N 21/41, G01M 3/38

(54) **OPTICAL FIBER GAS SENSOR**

(30) Priority: 19.05.2023 JP 2023083346
(71) Applicant: Cmiws Co., Ltd., Kyoto-shi, Kyoto 615-8203 (JP)
(72) Inventor: MASHIMO Kenji, Kyoto-shi Kyoto 615-8203 (JP)
(74) Representative: Grünecker Patent- und Rechtsanwälte PartG mbB
(86) International application number: PCT/JP2024/003189
(87) International publication number: WO 2024/241632

(57) **Abstract**

An optical fiber gas sensor according to the present invention includes a tubular case, an optical fiber having a fiber Bragg grating (FBG) part, a fixing member, a sensing member, a through hole, and a sealing member. The fixing member fixes the optical fiber in a state where the optical fiber is along an axial direction of an inner case and tension is applied to the FBG part. The FBG part is coated with the sensing member within the inner case. The sensing member is configured by a catalyst to accelerate a contact combustion reaction of detection object gas. The through hole is disposed within a wall of the inner case. The through hole is coated with the sealing member. The sealing member is configured by a hydrophobic film being permeable to the detection object gas.

## Description

### TECHNICAL FIELD

The present invention relates to an optical fiber gas sensor having a fiber Bragg grating (FBG).

### BACKGROUND ART

An electric-type gas sensor has been conventionally used to detect a gas leakage, and the like. However, for detection of flammable gas such as hydrogen gas, the electric-type gas sensor, which can be an ignition source, is required to apply an explosion-proof processing to use. Thus, a gas sensor having an optical fiber is proposed to solve such problem (for example, patent documents 1 and 2). The gas sensor having the optical fiber fails to be the ignition source, because wiring for power feeding and a measurement is unnecessary like the electric-type gas sensor. Further, such gas sensor is characterized in that many sensors can be disposed on measurement objects such as pipes.

The patent document 1 discloses a hydrogen gas sensor having a long-period fiber grating structure around a fiber core of an optical fiber where a gas sensing layer disposed all around a fiber cladding of the long-period fiber grating structure. In this structure, a hydrogen concentration is identified based on transmission power loss of the long-period fiber grating structure that varies when the gas sensing layer absorbs hydrogen. The patent document 2 discloses a hydrogen gas sensor having an FBG part formed in a core of an optical fiber where a platinum catalyst loaded on tungsten oxide film, which is a hydrogen sensitive substance, is arranged all around a cladding of the FBG part. In this structure, a concentration of hydrogen is identified based on a variation of light wavelength characteristics, when light transmits or reflects the FBG part as a result of heating or deforming during a reaction between the platinum catalyst loaded on tungsten oxide film and hydrogen.

### Citation List

### Patent Documents

Patent document 1: Japanese Unexamined Patent Application Publication No. 2009-244262
Patent document 2: Japanese Unexamined Patent Application Publication No. 2005-351651

### SUMMARY OF INVENTION

### Problems to Be Solved by Invention

According to the technologies disclosed in the patent document 1 and the patent document 2, the gas sensing layer to absorb detection object gas and the film made from the sensing substance to react with hydrogen are formed with uniform thickness all around the optical fiber in the long-period fiber grating structure or in a state of coating the FBG part. However, forming such film all around the optical fiber with the uniform thickness is not easy, thereby increasing in labor and man-hour, and cost. Further, when a plurality of gas detection parts is formed in a plurality of positions of the same optical fiber, the cost may be increased more.

The present invention is designed in view of such problems of the conventional arts, and an object of the present invention is to provide an optical fiber gas sensor, which can be relatively easily manufactured, capable of detecting detection object gas with high sensitivity.

### Solution to Problem

The present invention adopts following technical methods to attain the above-described object. First, an optical fiber gas sensor according to the present invention includes a tubular case, an optical fiber having a fiber Bragg grating (FBG) part, a fixing member, a sensing member, a through hole, and a sealing member. The fixing member fixes the optical fiber in a state where the optical fiber is along an axis direction of the case and tension is applied to the FBG part. The fiber Bragg grating part is coated with the sensing member within the case. The sensing member is configured by a catalyst for accelerating a contact combustion reaction of detection object gas. The through hole is disposed on a wall of the case. The through hole is coated with the sealing member. The sealing member is configured by a hydrophobic film that is permeable to detection object gas.

The optical fiber gas sensor according to the present invention has a structure where the FBG part of the optical fiber fixed within the case is coated with the sensing member, thereby being easily manufactured in comparison with a structure where the sensing member is disposed as a film having a constant thickness on all circumference of the optical fiber. Further, according to this structure, the optical fiber is fixed to the case, so that a Bragg wavelength of the FBG part can be significantly varied by deformation of the case caused by heat generated by a contact combustion reaction. Consequently, the detection object gas can be detected with high sensitivity.

In this optical fiber gas sensor, the inner case is configured by a material preferably having a thermal expansion coefficient larger than that of the optical fiber. A structure where the case is filled with the sensing member in a part of an area of the case in an axial direction can be adopted. For example, a platinum-supported silica catalyst can be used for the sensing member. A structure where a plurality of through holes is disposed in a position except for an area where the fiber Bragg grating is in contact with the case can be adopted. A structure where a cylindrical outer case for accommodating the case where the optical fiber is fixed is further disposed can be adopted. Furthermore, a structure where the optical fiber further has a FBG part for temperature compensation can be adopted.

### Effect of Invention

According to the present invention, the optical fiber gas sensor capable of detecting the detection object gas with high sensitivity can be relatively easily manufactured.

### BRIEF DESCRIPTION OF DRAWINGS

FIG. 1 is a schematic perspective view showing an example of an appearance of an optical fiber gas sensor in accordance with one embodiment of the present invention;
FIG. 2 is a longitudinal section view schematically showing an example of an inner structure of the optical fiber gas sensor in accordance with one embodiment of the present invention;
FIG. 3 shows an example of using the optical fiber gas sensor in accordance with one embodiment of the present invention;
FIG. 4 is a schematic perspective view showing an example of an appearance of another optical fiber gas sensor in accordance with one embodiment of the present invention;
FIG. 5 is a longitudinal section view schematically showing an example of an inner structure of another optical fiber gas sensor in accordance with one embodiment of the present invention;
FIG. 6 (a) to FIG. 6 (d) shows an example of measurement results of the optical fiber gas sensor in accordance with one embodiment of the present invention.

### DESCRIPTION OF EMBODIMENTS

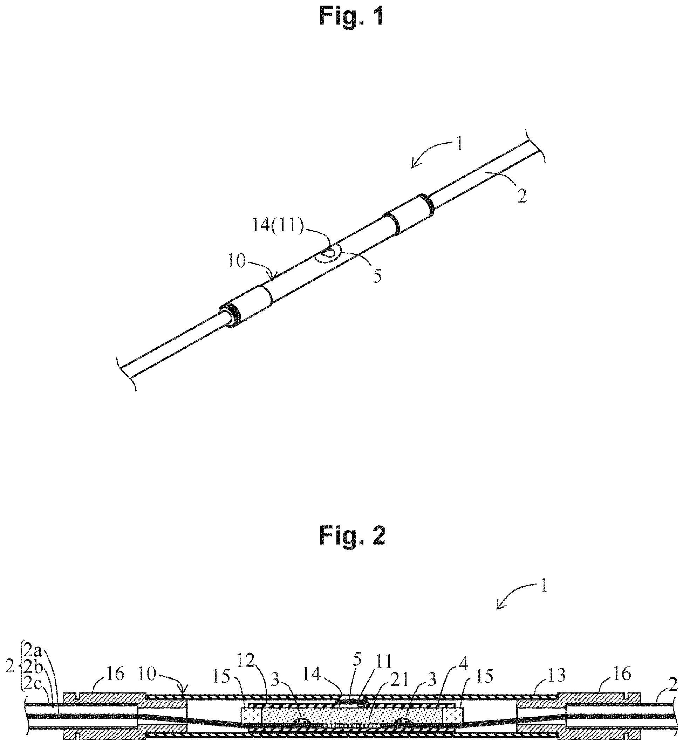

More detailed description of embodiments of the present invention is provided below with reference to the drawings. FIG. 1 is a schematic perspective view showing an appearance of an optical fiber gas sensor 1 according to an embodiment. FIG. 2 is a longitudinal section view showing an inner structure of the optical fiber gas sensor 1 according to the embodiment. FIG. 2 is the longitudinal section view along an axial direction of the optical fiber gas sensor 1 shown in FIG. 1.

As shown in FIG. 1 and FIG. 2, the optical fiber gas sensor 1 has a structure where an optical fiber 2 and a sensing member 4 are disposed within a tubular double case 10. Herein, the cylindrical double case 10, but is not particularly limited to, is illustrated. The double case 10 is configured by an inner case 12 and an outer case 13. The optical fiber 2 and the sensing member 4 are disposed within the inner case 12. A material of the inner case 12 and the outer case 13 is not specifically limited, but the material of the inner case 12 preferably has a thermal expansion coefficient larger than that of the optical fiber 2. In this example, a material of the double case 10 is stainless steel in this embodiment. An outer diameter of the double case 10 is approximately 5 mm, but is not particularly limited.

The optical fiber 2 has a core 2a to transmit light, a cladding 2b which surrounds the core to reflect transmitted light towards the core, and a resin jacket 2c which surrounds the cladding to protect the core and the cladding, and has a structure where the core 2a, the cladding 2b, and the resin jacket 2c are disposed in order from a center. The optical fiber 2 has a fiber Bragg grating (FBG) part 21. As known, the FBG part 21 reflects light of a wavelength regulated by Bragg wavelength. The FBG part 21 is configured by a plurality of diffraction gratings formed at a predetermined interval in the core of the optical fiber 2. The Bragg wavelength is proportional to a product of a refractive index of the optical fiber and the interval disposition between the diffraction gratings. Thus, because of an increase in the reflective index caused by a temperature rise and an expansion of the interval between the diffraction gratings caused by stretching the optical fiber 2, the optical wavelength reflected by the FBG part 21 becomes larger. Because of a decrease in the reflective index caused by a temperature fall and a narrowing of the interval between the diffraction gratings caused by compressing the optical fiber 2, the optical wavelength reflected by the FBG part 21 becomes smaller. The FBG part 21 is shown in black-and-white stripes in the drawings for convenience.

As shown in FIG. 2, the inner case 12 is accommodated within the outer case 13. An outer diameter of the inner case 12, but is not particularly limited to, is slightly smaller than an inner diameter of the outer case 13. The optical fiber 2 is disposed along an axis direction of the inner case 12, and both sides of the FBG part 21 in the optical fiber 2 are fixed to an inner wall face of the case 10 with a fixing member 3 such as an adhesive agent in a state where the FBG 21 is in contact with an inner wall face of the inner case 12. When the optical fiber 2 is fixed, tension (pretension) is applied to the FBG part 21. A fixing member 3, which is an adhesive agent applied in a spot shape, is used here, but is not particularly limited thereto.

The FBG part 21 is coated with the sensing member 4 within the inner case 12. The sensing member 4 is configured by a catalyst to accelerate a contact combustion reaction of detection object gas. The sensing member 4 can be selected accordingly depending on a kind of detection object gas. For example, when the detection object gas is hydrogen, a platinum-supported silica catalyst can be used as the sensing member 4. In this case, the sensing member 4 is disposed in a state where the FBG part 21 is coated with the sensing member 4 and the inner part of the inner case 12 (a part of an area in the axial direction) is filled with the sensing member 4, but is not particularly limited thereto.

Opening ends of the inner case 12 are filled with the sensing member 4, and subsequently the opening ends are sealed with a resin-made sealing plug 15. Accordingly, leakage of the sensing member 4 to outside of the inner case 12 can be prevented. Opening ends of the outer case 13 are sealed with one-touch couplers 16. The one-touch coupler 16 clamps the optical fiber 2 (the resin jacket 2c), thereby having a function to fix the optical fiber 2 to the double case 10.

The inner case 12 has a through hole 11. In this embodiment, the through hole 11 is round shaped and is disposed in a position opposite to the FBG part 21 fixed to the inner case 12, but is not particularly limited thereto. The number, a size, and/or a shape of the through hole 11 are not particularly limited. For example, a plurality of through holes can be disposed in positions except for the position where the FBG part 21 is in contact with the inner case 12. The outer case 13 has a corresponding through hole 14 in a position overlapping with the through hole 11 in a state where the inner case 12 is accommodated within the outer case 13. The through hole 14 is round shaped and has the same diameter as the through hole 11 has, but is not particularly limited thereto. The through hole 11 in the inner case 12 is coated with a sealing member 5. The sealing member 5 is configured by a hydrophobic film that is permeable to the detection object gas. For example, when the detection object gas is hydrogen, polytetrafluoroethylene (PTFE) can be used as the sealing member 5.

In the optical fiber gas sensor 1 having the above structure, in an atmosphere where detection object gas exists, the detection object gas intrudes into the inner case 12 through the through hole 14, the sealing member 5, and the through hole 11, and is contact with the sensing member 4. At this time, the sensing member 4 generates heat by a contact combustion reaction. Because the heat is transmitted to the inner case 12 and/or the sensing member 4 is expanded by the heat, the inner case 12 is deformed by stretching the inner case 12.

As described above, because the FBG part 21 is fixed to the inner case 12 in a state where both sides of the FBG part 21 are in contact with the inner case 12, a Bragg wavelength of the FBG part 21 becomes larger when the inner case 12 is deformed. Heat quantities generated by the contact combustion reaction of the sensing member 4 increase in response to an increase in a concentration of the detection object gas. Thus, a deformation amount of the case 10 increases in response to the increase in the concentration of the detection object gas. Accordingly, a corresponding relation between a shift amount of the Bragg wavelength of the FBG part 21 and the concentration of the detection object gas is acquired beforehand, and thereby the concentration of the detection object gas can be identified by the shift amount of the Bragg wavelength of the FBG part 21.

In this embodiment, since the inner case 12 is accommodated within the outer case 13 without being fixed to the outer case 13, in case an external force from outside is applied to the double case 10 during a detection operation, the deformation of the inner case 12 caused by the external force can be suppressed. Namely, a false detection caused by the external force can be suppressed.

As described above, the optical fiber gas sensor 1 has a structure where the FBG part 21 of the optical fiber 2 fixed to the inner wall face of the inner case 12 is coated with the sensing member 4, thereby being easily manufactured in comparison with a structure where the sensing member is disposed as a film having a constant thickness on all circumference of the optical fiber 2. Further, according to the structure of this embodiment, because the FBG part 21 is in contact with the inner case 12, the Bragg wavelength of the FBG part 21 is significantly varied by the deformation of the case caused by the heat generated by the contact combustion reaction. Consequently, the detection object gas can be detected with high sensitivity.

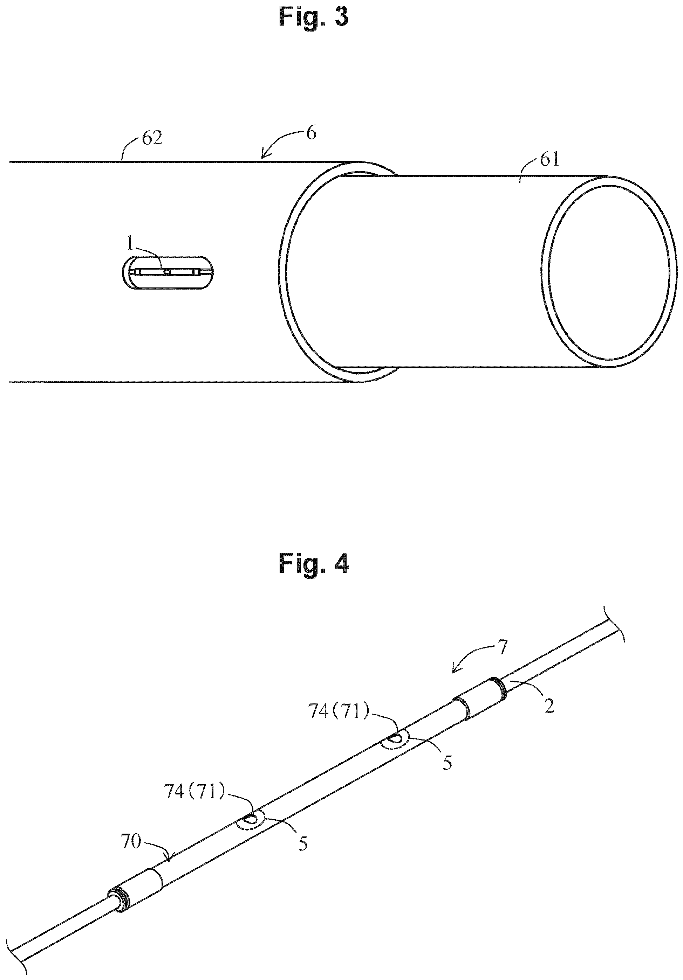

Here, a usage example of the optical fiber gas sensor 1 is described. FIG. 3 illustrates the usage example of the optical fiber gas sensor. FIG. 3 illustrates the usage example of a gas transport tube for transporting gas.

As shown in FIG. 3, a gas transport tube 6 has a double tube structure configured by an inner tube 61 and an outer tube 62 which accommodates the inner tube 61. In this structure, when gas leakage from the inner tube 61 occurs, the leaked gas is held in the outer tube 62 so that gas leakage to outside can be prevented.

In such the gas transport tube 6, the optical fiber sensor 1 can be installed on an outer wall face of the inner tube 61 and/or an inner wall face of the outer tube 62. In FIG. 3, the outer tube 62 has an opening to expose the optical fiber gas sensor 1 for illustration. The outer tube 62 has no such opening in an actual usage state. As known, one optical fiber 2 having a plurality of FBG parts 21 enables multipoint detection. In other words, when the optical fiber 2 having the plurality of FBG parts 21 is installed along the axial direction of the gas transport tube 6, information of a position whereabout a leakage occurs can be obtained.

As described above, the Bragg wavelength is defined by the refractive index and the grating interval between the diffraction gratings of the optical fiber 2. Thus, the Bragg wavelength varies with a variation of the refractive index due to a change in temperature, or with expansion and contraction of the optical fiber. In other words, when an abrupt temperature change occurs during the detection operation, it may not be unable to determine whether the variation of the Bragg wavelength occurs due to the deformation of the inner case 12 or the temperature change.

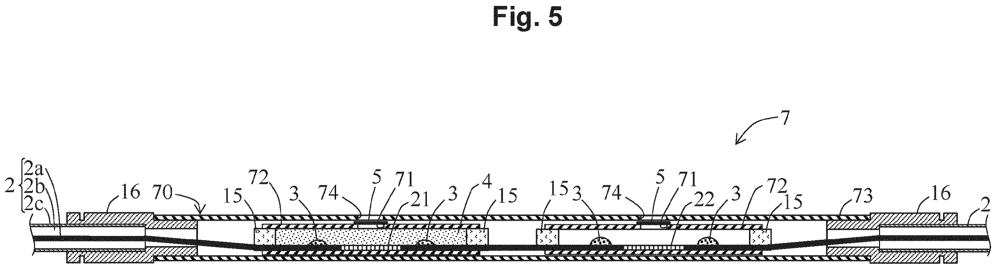

As a measure for the above, an FBG part for temperature compensation can be disposed. FIG. 4 is a schematic perspective view showing an appearance of another optical fiber gas sensor 7 in accordance with this embodiment. FIG. 5 is a longitudinal section view showing an internal structure of another optical fiber gas sensor 7 in accordance with this embodiment. FIG.5 is the longitudinal section view along an axial direction of the optical fiber gas sensor 7 shown in FIG. 4.

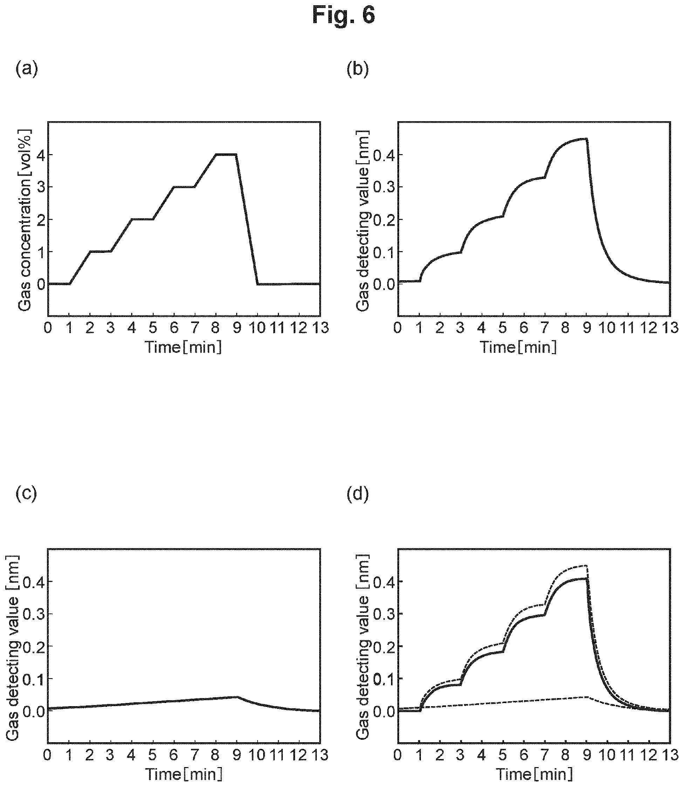

As shown in FIG. 5 and FIG. 6 (a) to FIG. 6 (d), a structure of the optical fiber gas sensor 7 differs from that of the optical fiber gas sensor 1 on the point that the optical fiber gas sensor 7 has the additional FBG part 22 for temperature compensation. Other structures of the optical fiber gas sensor 7 are the same as that of the optical fiber gas sensor 1, and the same reference signs are used for the components having the same functions and effects.

As shown in FIG. 4 and FIG. 5, the optical fiber gas sensor 7 has a cylindrical double case 70 where two inner cases 72 are accommodated within one outer case 73. An outer diameter of the inner case 72, but is not particularly limited to, is slightly smaller than an inner diameter of the outer case 73. The FBG part 21 is disposed in an inner part of one inner case 72. The FBG part 22 for temperature compensation is disposed in an inner part of another inner case 72. In the same manner as the optical fiber gas sensor 1, the optical fiber 2 is disposed along the axis direction of each of the inner cases 72, and portions of the optical fiber 2 in both sides of the FBG part 21 are fixed to an inner wall face of the corresponding inner case 72 with the fixing member 3 such as the adhesive agent in a state where the FBG part 21 is in contact with an inner wall face of the inner case 72. Further, portions of the optical fiber 2 in both sides of the FBG part 22 are fixed to the inner wall of the corresponding inner case 72 with the fixing member 3 such as the adhesive agent in a state where the FBG part 22 is in contact with the inner wall face of the inner case 72.

In this example, the FBG part 21 and the FBG part 22 are adjacently formed in the same optical fiber 2. Here, the FBG part 21 and the FBG part 22 are set to have different Bragg wavelengths. Such structure can be attained by, for example, forming the FBG parts having the same Bragg wavelengths adjacently in the same optical fiber 2, and by applying different pretensions to the FBG part 21 and the FBG part 22 when each of the FBG parts 21 and 22 is fixed to the corresponding inner case 72 with the fixing member 3. In this embodiment, the FBG part 21 and the FBG part 22 are respectively fixed to the different inner cases 72, so that such different pretensions can be easily applied to the FBG part 21 and the FBG part 22.

Each of the inner cases 72 has a through hole 71. In this embodiment, the through holes 71 are round shaped and are disposed in positions opposite to the FBG part 21 and the FBG part 22 which are fixed to the inner case 72, but are not particularly limited thereto. The number, a size, and/or a shape of the through holes 71 are not particularly limited. For example, a plurality of the through holes 71 can be disposed in positions except for the positions where the FBG parts 21 and 22 are in contact with the inner cases 72. The outer case 73 has corresponding through holes 74 in positions overlapping with the through holes 71 in a state where the inner cases 72 are accommodated within the outer case 73. The through holes 74 are round shaped and have the same diameter as those of the through holes 71, but are not particularly limited thereto. The through holes 71 on the inner cases 72 are coated with the sealing members 5 in the same manner as the optical gas fiber sensor 1.

In the optical fiber gas sensor 7, the FBG part 21 is coated with the sensing member 4, but the FBG part 22 is not coated with the sensing member 4. Thus, the optical fiber gas sensor 7 is installed in an atmosphere where the detection object gas exists, the FBG part 21 is affected by heat generated by a contact combustion reaction of the sensing member 4, but the FBG part 22 is not affected by the heat. That is, the FBG part 22 is only affected by an ambient temperature. In this example, the FBG part 22 is exposed without any coating member, but may be coated with any other members. For example, the FBG part 22 may be coated with a member that has a similar thermal expansion coefficient as the sensing member 4 and generates no contact combustion reaction with the detection object gas.

In the optical fiber gas sensor 7 having the above structure, in the atmosphere where the detection object gas exists, the detection object gas intrudes into the inner case 72 through the through hole 74, the sealing member 5, and the through hole 71, and is contact with the sensing member 4. As a result, as described above, the Bragg wavelength of the FBG part 21 is shifted. When the ambient temperature varies, a wavelength shift amount caused by the temperature variation is superimposed on the Bragg wavelength shift amount of the FBG part 21. However, the wavelength shift amount caused by the temperature variation can be obtained as the wavelength shift amount of the FBG part 22. Therefore, the wavelength shift amount excluding the effect of the ambient temperature can be obtained by calculating a difference between the shift amount of the FBG part 21 and the shift amount of the FBG part 22.

FIG. 6 (a) to FIG. 6 (d) shows an example of a measurement result of the optical fiber gas sensor 7. FIG. 6 (a) shows time variations of a concentration of the detection object gas (hydrogen) flew into an ambient atmosphere of the optical fiber gas sensor 7. FIG. 6 (b) shows time dependency on the concentration of the detection object gas which is identified based on the shift amount of the Bragg wavelength of the FBG part 21. FIG. 6 (c) shows time dependency of values of the concentration of the detection object gas in the ambient atmosphere which is converted from the shift amount of the Bragg wavelength of the FBG part 22. FIG. 6 (d) shows time dependency of the concentration of the detection object gas in the ambient atmosphere which is identified based on the shift amount of the Bragg wavelength of the FBG part 21 and the shift amount of the Bragg wavelength of the FBG part 22. In FIG. 6 (d), broken lines show the data of FIG. 6 (b) and FIG. 6 (c) for reference. It can be understood that FIG. 6 (a) to FIG. 6 (d) shows that the optical fiber gas sensor 7 can successfully detect the concentration of the detection object gas.

As described above, according to the present invention, the optical fiber gas sensor capable of detecting the detection object gas with high sensitivity can be comparatively easily manufactured.

The above-described embodiments do not limit the technical scope of the present invention, and, other than those already described above, various modifications and applications are possible within the scope of the present invention. For example, in the above-described embodiments, the examples of the FBG parts 21 and 22 that are fixed to the inner wall faces in contact with the inner cases 12 and 72 is illustrated as the particularly preferred embodiments, but both sides of the FBG parts 21 and 22 may be fixed to the inner cases 12 and 72, and are not required to be fixed in contact with the inner wall faces. Further, in the above-described embodiments, the examples of the inner cases 12 and 72 that are accommodated within the outer cases 13 and 73 are illustrated as the particularly preferred embodiments, but a structure without the outer case can be adopted. Further, in the above-described embodiments, the through holes 11 and 71 that are disposed in the positions except for the regions where the FBG parts 21 and 22 are in contact with the inner cases 12 and 72 are illustrated as the particularly preferred embodiments, but can be disposed in the regions where the FBG parts 21 and 22 are in contact with the inner cases 12 and 72. Furthermore, the form of the cases, and the size, the number and the disposition of the fixing members, and the like are merely exemplified, and can be changed as required. Meanwhile, the physical shapes of each of the above-described components can be changed as required within a range to obtain the effect of the present invention.

### Industrial Applicability of Invention

According to the present invention, the optical fiber gas sensor capable of detecting the detection object gas with high sensitivity can be comparatively easily manufactured, thereby being useful as the optical fiber gas sensor.

### Description of Reference Signs

1 optical fiber gas sensor
2 optical fiber
3 fixing member
4 sensing member
5 sealing member
10, 17 double case
11, 14, 71, 74 through hole
12, 72 inner case (case)
24, 73 outer case
21 FBG part
22 FBG part (for temperature compensation)

## Claims

1. An optical fiber gas sensor, comprising:
a tubular case;
an optical fiber having a fiber Bragg grating part;
a fixing member for fixing the optical fiber in a state where the optical fiber is along an axis direction of the case and tension is applied to the fiber Bragg grating part;
a sensing member, configured by a catalyst for accelerating a contact combustion reaction of detection object gas, for coating the fiber Bragg grating part within the case;
a through hole disposed on a wall of the case; and
a sealing member, being hydrophobic and permeable to the detection object gas, for coating the through hole.

2. The optical fiber gas sensor according to claim 1, wherein the case is configured by a material having a thermal expansion coefficient larger than the thermal expansion coefficient of the optical fiber.

3. The optical fiber gas sensor according to claim 2, wherein a part of an area in the axial direction of the case is filled with the sensing member.

4. The optical fiber gas sensor according to claim 3, wherein the sensing member is a platinum-supported silica catalyst.

5. The optical fiber gas sensor according to claim 4, wherein a plurality of through holes is disposed in a position except for an area where the fiber Bragg grating part is in contact with the case.

6. The optical fiber gas sensor according to any one of claims 1 to 5, further comprising a cylindrical outer case for accommodating the case.

7. The optical fiber gas sensor according to claim 6, wherein the optical fiber further comprises a fiber Bragg grating part for temperature compensation.
